# FASCICULE DE BREVET EUROPEEN

(11) **EP 4 565 118 B1**
(45) Date de publication et mention de la délivrance du brevet: **18.02.2026**
(21) Numéro de dépôt: 23738492.0
(22) Date de dépôt: 03.07.2023
(51) Int. Cl.: A61B 5/00, A61B 8/08

(54) **DISPOSITIF DE PROJECTION POUR OUTIL DE LOCALISATION D'UN VAISSEAU SANGUIN**
PROJEKTIONSVORRICHTUNG FÜR EIN WERKZEUG ZUR LOKALISIERUNG EINES BLUTGEFÄSSES
PROJECTION DEVICE FOR A TOOL FOR LOCATING A BLOOD VESSEL

(30) Priorité: 01.08.2022 FR 2207967
(43) Date de publication de la demande: 11.06.2025
(73) Titulaire: Arterya, 14200 Herouville-Saint-Clair (FR)
(72) Inventeur: CALLOCH, Benjamin, 14000 Caen (FR); LEGARREC, Laurine, 14000 Caen (FR); DERLY, Lucile, 14000 CAEN (FR)
(74) Mandataire: HGF
(86) Numéro de dépôt international: PCT/EP2023/068262
(87) Numéro de publication internationale: WO 2024/028014

(56) Documents cités:
- US-A1- 2007 161 907
- US-A1- 2011 125 028
- US-A1- 2015 011 887
- US-A1- 2016 066 791
- US-A1- 2021 220 059
- US-A1- 2021 228 151

## Description

### Domaine technique de l'invention

La présente invention concerne de manière générale les techniques permettant la localisation de vaisseaux sanguins, et en particulier les dispositifs correspondants.

### Arrière-plan technique

On appelle « gaz du sang » l'analyse des gaz du sang (ou gazométrie sanguine). Lors d'une telle analyse, le praticien mesure l'acidité du sang et les quantités d'oxygène et de dioxyde de carbone dans le sang. Le sang est préférentiellement prélevé dans une artère.

L'examen permet d'évaluer les échanges pulmonaires et de détecter les modifications des concentrations d'oxygène et de dioxyde de carbone dans le sang artériel et notamment dans le sang qui se dirige vers les tissus. En effet, par exemple, lorsque le sang passe à travers les poumons, il s'enrichit en oxygène et s'appauvrit en dioxyde de carbone.

L'analyse de gaz du sang permet aussi d'évaluer l'équilibre acido-basique d'un patient.

Lors de l'examen, une prise de sang au niveau d'une artère est effectuée. En général il s'agit de l'artère radiale (poignet), humérale (bras) ou fémorale (aine). Une fois le prélèvement effectué, il faut placer une compresse de gaze ou de coton et compresser le point de ponction fermement pendant quelques minutes.

Par conséquent, il serait avantageux de déterminer avec précision l'emplacement de l'artère par des moyens non invasifs avant la prise de sang, et de fournir au praticien une indication visuelle de l'emplacement de l'artère pour faciliter la prise de sang.

En particulier, il serait intéressant d'afficher l'emplacement de l'artère sur une longueur suffisante pour permettre au praticien de déterminer l'orientation de l'artère, de sorte que l'aiguille ou le stylet puisse être positionné pour couper l'artère le long de son axe. Il est généralement souhaitable de croiser l'artère à un angle compris entre environ 30 degrés et 45 degrés. Lorsque l'artère est convenablement localisée, le praticien peut alors aligner l'aiguille à un angle approprié pour l'insertion dans le vaisseau sanguin.

Des systèmes de l'art antérieur, par exemple les systèmes pour localiser un vaisseau sanguin, ont été décrits.

Par exemple, la demande de brevet américain N0. US2018325448A1 divulgue un dispositif de localisation d'artère qui comprend un réseau de capteurs configuré pour être attaché à une surface cutanée recouvrant une artère cible. Le réseau de capteurs est un réseau de détecteurs, par exemple des détecteurs de pression configurés pour générer des signaux sensibles à une pression ou à un changement de pression. Un dispositif d'affichage est disposé sur le réseau de capteurs. Un circuit contrôleur est configuré pour recevoir des signaux générés par le réseau de capteurs, pour identifier à partir des signaux reçus des impulsions de pression périodiques qui ont une fréquence dans une plage de fréquences prédéterminée correspondant à une fréquence pulsatile et qui définissent un chemin allongé à travers au moins une partie de la réseau de capteurs, et pour afficher sur le dispositif d'affichage une image qui recouvre le chemin allongé à travers le réseau de capteurs, de sorte que l'affichage montre une projection de la position bidimensionnelle de l'artère sous l'affichage. US 2018/325448 A1 décrit un autre dispositif de localisation d'un vaisseau sanguin.

Cependant, un tel dispositif de localisation à base d'un réseau de capteurs est relativement encombrant et ne présente pas une précision suffisante pour détecter des artères dans toutes les conditions d'utilisation du dispositif. En outre, un tel dispositif n'est pas aisé et rapide à installer sur un patient.

### Résumé de l'invention

Sur la base de ce problème, la présente invention a donc pour tâche de développer un dispositif de localisation d'un vaisseau sanguin du type précédemment indiqué de façon à garantir une plus grande précision. L'invention selon certains de ses modes de réalisations, a également pour objectif de fournir un tel dispositif de localisation qui est moins encombrant que certains dispositifs de l'art antérieur. L'invention, selon certains de ses modes de réalisations, a également pour objectif de fournir un tel dispositif de localisation qui indique à l'utilisateur qui peut être un soignant ou un praticien un point sur lequel il doit effectuer une opération (tel qu'un prélèvement de sang). L'invention selon certains de ses modes de réalisations, a également pour objectif de fournir un tel dispositif qui soit aisé et rapide à installer sur un patient. L'invention selon certains de ses modes de réalisations, a également pour objectif de fournir un tel dispositif qui soit simple à utiliser. L'invention selon certains de ses modes de réalisations, a également pour objectif de fournir un tel dispositif qui fournisse simplement à l'utilisateur plus d'informations de localisation que les dispositif classiques.

En ce qui concerne les dispositifs de localisation de vaisseaux sanguins, la tâche sur laquelle se base l'invention est résolue par l'objet de la présente invention, des développements avantageux du dispositif de localisation selon l'invention étant spécifiés ci-après.

Par conséquent, l'invention concerne en particulier un dispositif de localisation d'un vaisseau sanguin d'un membre d'un être vivant configuré pour être disposé sensiblement en regard d'une surface de peau recouvrant le vaisseau sanguin dudit membre, le dispositif comprenant des moyens de détermination de la localisation du vaisseau sanguin. Selon l'invention, le dispositif de localisation comprend des moyens de projection sur la surface de peau d'au moins une image de localisation du vaisseau sanguin.

Le dispositif de localisation selon l'invention peut être utilisé pour localiser tout type de vaisseau sanguin tel que par exemple des veines, des capillaires ou des artères. Il peut être avantageusement utilisé sur des êtres humains mais peut également être utilisé sur des animaux (applications vétérinaires).

Préférentiellement, le dispositif de localisation selon l'invention est utilisé pour localiser une artère dans le cadre d'une prise de sang artérielle.

Selon l'invention, ladite au moins une image de localisation du vaisseau sanguin comprend au moins une des images suivantes :
- une information représentative de la profondeur du vaisseau sanguin dans ledit membre ;
- une représentation d'une portion du vaisseau sanguin.

Selon un mode de réalisation de l'invention, ladite au moins une image est projetée sur la surface de peau de manière à ce qu'elle(s) se situe(nt) sensiblement à l'aplomb du vaisseau sanguin.

Selon un mode de réalisation de l'invention, les moyens de projection comprennent au moins un projecteur et un miroir inclinable.

Selon un mode de réalisation de l'invention, les moyens de détermination de la localisation du vaisseau sanguin comprennent au moins un émetteur d'ondes acoustiques configuré pour émettre des ondes acoustiques - ci-après onde acoustiques émises - dans le membre à travers la surface de peau, au moins un récepteur d'ondes acoustiques configuré pour recevoir les ondes acoustiques réfléchies par le membre - ci-après ondes acoustiques réfléchies, un module de mesure d'un paramètre des ondes acoustiques réfléchies ou d'une différence de valeur d'un paramètre entre les ondes acoustiques réfléchies et les ondes acoustiques émises et un module d'obtention de la localisation du vaisseau sanguin en fonction dudit paramètre ou de ladite différence de valeur dudit paramètre.

Selon un mode de réalisation de l'invention, le module de mesure est un module de mesure du déphasage entre les ondes acoustiques réfléchies et les ondes acoustiques émises et le module d'obtention de la localisation du vaisseau sanguin tient compte dudit déphasage mesuré.

Selon un mode de réalisation de l'invention, le module de mesure est un module de mesure de l'amplitude des ondes acoustiques réfléchies et le module d'obtention de la localisation du vaisseau sanguin tient compte de ladite amplitude.

Selon un mode de réalisation de l'invention, le module de mesure est un module de mesure de la différence de fréquence entre les ondes acoustiques réfléchies et les ondes acoustiques émises et le module d'obtention de la localisation du vaisseau sanguin tient compte de ladite différence de fréquence.

Selon un mode de réalisation de l'invention, les moyens de détermination de la localisation du vaisseau sanguin comprennent au moins une première matrice d'émetteurs et de récepteurs d'ondes acoustiques située en une première zone du dispositif et une seconde matrice d'émetteurs et de récepteurs d'ondes acoustiques située en une seconde zone du dispositif.

Selon un mode de réalisation de l'invention, le module de mesure d'un paramètre ou d'une différence de valeur d'un paramètre comprend des moyens de mesure d'une première valeur de paramètre ou de différence de valeur de paramètre au niveau de la première zone et des moyens de mesure d'une seconde valeur de paramètre ou de différence de valeur de paramètre au niveau de la seconde zone. Selon un mode de réalisation de l'invention, une image de localisation du vaisseau sanguin est construite à partir d'une première localisation déterminée à partir de la première valeur de paramètre ou de différence de valeur de paramètre et d'une seconde localisation déterminée à partir de la seconde valeur de paramètre ou de différence de valeur de paramètre.

Selon un mode de réalisation de l'invention, le dispositif de localisation comprend une première branche comprenant les moyens de détermination de la localisation du vaisseau sanguin, ladite première branche étant prévue pour être positionnée de sorte à mettre en regard les moyens de détermination de la localisation et la surface de peau recouvrant le vaisseau sanguin dudit membre et une seconde branche prévue pour maintenir la première branche contre ledit membre. Selon un mode de réalisation de l'invention, la première branche comprend un premier doigt comprenant la première matrice et un second doigt comprenant la seconde matrice.

La seconde branche peut être rigide (par exemple réalisé en matière plastique ou en métal) ou plastique ou même élastique. Cela peut par exemple être une lanière élastique.

Selon un mode de réalisation de l'invention, le dispositif de localisation est une pince adaptée pour être disposée sur le membre d'un être vivant.

Selon un mode de réalisation de l'invention, la pince comprend des moyens de mise en mouvement relatif de la première branche par rapport à la seconde branche.

Selon l'invention, le dispositif de localisation comprend des moyens de détection d'un déplacement du vaisseau sanguin. Selon un mode de réalisation de l'invention, les moyens de détermination de la localisation du vaisseau sanguin et les moyens de projection d'au moins une image de localisation sont activés par les moyens de détection d'un déplacement du vaisseau sanguin.

L'invention selon au moins un de ses modes de réalisation concerne également un procédé de localisation d'un vaisseau sanguin d'un membre d'un être vivant configuré pour être disposé sensiblement en regard d'une surface de peau recouvrant le vaisseau sanguin dudit membre, le procédé comprenant une étape de détermination de la localisation du vaisseau sanguin et une étape de projection sur la surface de peau d'au moins une image de localisation du vaisseau sanguin.

Selon l'invention, ladite au moins une image de localisation du vaisseau sanguin comprend au moins une des images suivantes :
- une information représentative de la profondeur du vaisseau sanguin dans ledit membre ;
- une représentation d'une portion du vaisseau sanguin.
- une représentation d'une portion du vaisseau sanguin.

Selon un mode de réalisation de l'invention, ladite au moins une image est projetée sur la surface de peau de manière à ce qu'elle(s) se situe(nt) sensiblement à l'aplomb du vaisseau sanguin.

Selon un mode de réalisation de l'invention, l'étape de projection met en œuvre au moins un projecteur et un miroir inclinable.

Selon un mode de réalisation de l'invention, l'étape de détermination de la localisation du vaisseau sanguin comprend :
- une étape d'émission d'ondes acoustiques - ci-après onde acoustiques émises - dans le membre à travers la surface de peau par au moins un émetteur d'ondes acoustiques configuré pour émettre des ondes acoustiques et une étape de réception d'ondes acoustiques réfléchies par le membre - ci-après ondes acoustiques réfléchies, par au moins un récepteur d'ondes acoustiques configuré pour recevoir des ondes acoustiques ;
- une étape de mesure d'un paramètre des ondes acoustiques réfléchies ou d'une différence de valeur d'un paramètre entre les ondes acoustiques réfléchies et les ondes acoustiques émises et
- une étape d'obtention de la localisation du vaisseau sanguin en fonction dudit paramètre ou de ladite différence de valeur dudit paramètre.

Selon un mode de réalisation de l'invention, l'étape de mesure est une étape de mesure du déphasage entre les ondes acoustiques réfléchies et les ondes acoustiques émises et l'étape d'obtention de la localisation du vaisseau sanguin tient compte dudit déphasage mesuré.

Selon un mode de réalisation de l'invention, l'étape de mesure est une étape de mesure de l'amplitude des ondes acoustiques réfléchies et l'étape d'obtention de la localisation du vaisseau sanguin tient compte de ladite amplitude.

Selon un mode de réalisation de l'invention, l'étape de mesure est une étape de mesure de la différence de fréquence entre les ondes acoustiques réfléchies et les ondes acoustiques émises et l'étape d'obtention de la localisation du vaisseau sanguin tient compte de ladite différence de fréquence.

Selon un mode de réalisation de l'invention, l'étape de détermination de la localisation du vaisseau sanguin met en œuvre au moins une première matrice d'émetteurs et de récepteurs d'ondes acoustiques située en une première zone et une seconde matrice d'émetteurs et de récepteurs d'ondes acoustiques située en une seconde zone.

Selon un mode de réalisation de l'invention, l'étape de mesure d'un paramètre ou d'une différence de valeur d'un paramètre comprend une étape de mesure d'une première valeur de paramètre ou de différence de valeur de paramètre au niveau de la première zone et une étape de mesure d'une seconde valeur de paramètre ou de différence de valeur de paramètre au niveau de la seconde zone. Selon un mode de réalisation de l'invention, une image de localisation du vaisseau sanguin est construite à partir d'une première localisation déterminée à partir de la première valeur de paramètre ou de différence de valeur de paramètre et d'une seconde localisation déterminée à partir de la seconde valeur de paramètre ou de différence de valeur de paramètre.

Selon l'invention, le procédé de localisation d'un vaisseau sanguin comprend une étape de détection d'un déplacement du vaisseau sanguin. Selon l'invention, la détection d'un déplacement du vaisseau sanguin active une étape de détermination de la localisation du vaisseau sanguin et une étape de projection sur la surface de peau d'au moins une image de localisation du vaisseau sanguin.

Préférentiellement, chaque émetteur et/ou récepteur d'ondes acoustiques peut être reconfiguré respectivement en un récepteur et/ou un émetteur d'ondes acoustiques.

Préférentiellement, les émetteurs et les récepteurs d'onde acoustiques comprennent des éléments piézoélectriques.

Préférentiellement, le module de mesure d'un paramètre ou d'une différence de valeur d'un paramètre comprend un filtre et un amplificateur.

L'invention concerne également une pince adaptée pour être disposée sur un membre d'un être vivant.

Préférentiellement, le vaisseau sanguin est une artère, l'être vivant un être humain et le membre un poignet.

Préférentiellement, la pince comprend une matrice réalisée en silicone. L'invention concerne en outre un procédé pour la fabrication d'un dispositif de localisation d'un vaisseau sanguin selon l'invention.

### Brève description des figures

D'autres caractéristiques et avantages de l'invention apparaitront au cours de la lecture de la description détaillée qui va suivre pour la compréhension de laquelle on se reportera aux dessins annexés dans lesquels :
[Fig. 1] montre schématiquement une vue de dessus d'une pince selon un mode de réalisation de l'invention ;
[Fig. 2] montre schématiquement les moyens de détermination de la localisation de l'artère de la pince de la figure 1 installée sur le poignet et le cheminement des ondes acoustiques émises et réfléchies ;

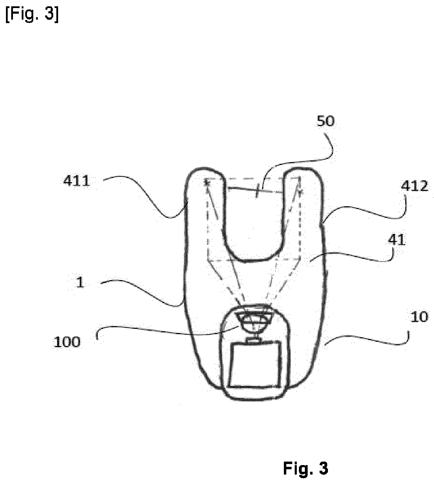

[Fig. 3] montre schématiquement une vue de dessus de la pince de la figure 1 ;
[Fig. 4] montre est schéma bloc du circuit électronique des moyens de localisation de la pince de la figure 1.

### Description détaillée de l'invention

Différents aspects de différents modes de réalisation d'un dispositif de localisation d'un vaisseau sanguin et d'un procédé de localisation d'un vaisseau sanguin sont décrits plus en détail ci-dessous, en faisant référence aux dessins joints.

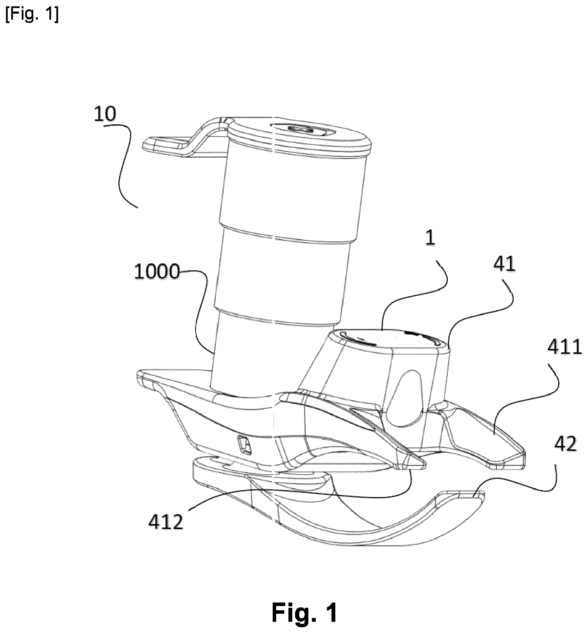

La figure 1 montre schématiquement un mode de réalisation exemplaire d'un dispositif de localisation 10 d'un vaisseau sanguin selon l'invention.

Selon ce mode de réalisation de l'invention, le dispositif de localisation 10 comprend une première branche 41 comprenant des moyens de détermination de la localisation 1 du vaisseau sanguin, ladite première branche 41 étant prévue pour être positionnée de sorte à mettre en regard les moyens de détermination de la localisation 1 et la surface de peau recouvrant le vaisseau sanguin du membre et une seconde branche prévue 42 pour maintenir la première branche 41 contre le membre.

Dans un dispositif de localisation selon des modes de réalisation de l'invention, au moins une ou chacune des première et seconde branche peut comprendre au moins deux brins articulés ou solidarisés l'un à l'autre.

Selon des modes de réalisation de l'invention, les deux branches peuvent être réalisés en un matériau plastique, en PVC, en silicone, en cuir, en nylon, ou en toute autre matière synthétique ou naturelle. Les deux branches peuvent s'attacher l'un à l'autre en leur extrémité libre (celle opposée à l'extrémité en contact avec les moyens de mise en mouvement) grâce à tout mécanisme de solidarisation (par exemple bouton poussoir, boucle avec pin métallique, ...). Bien entendu, les branches peuvent également ne pas s'attacher l'une à l'autre en leur extrémité libre (celle opposée à l'extrémité en contact avec les moyens de mise en mouvement) tel qu'illustré par la figure 1.

La seconde branche 42 peut être rigide (par exemple réalisé en matière plastique ou en métal) ou plastique ou même élastique. Dans des variantes du présent mode de réalisation, la seconde branche 42 peut par exemple être une lanière élastique ou une lanière en cuir qui est solidarisée à la première branche et qui maintien la première branche contre le membre.

Par exemple, le dispositif de localisation 10 est une pince 10 (telle qu'illustrée par la figure 1) adaptée pour être disposée sur le membre d'un être vivant.

Bien entendu, selon des variantes du présent mode de réalisation, un dispositif de localisation peut ne pas être une pince. Il peut par exemple présenter une première branche 41 telle que décrite ci avant et ci après et une lanière (ou portion de bracelet) en cuir ou en matière élastique pour maintenir la première branche 41 contre le membre.

Dans le présent mode de réalisation de l'invention, le dispositif de localisation 10 est par exemple utilisé pour détecter et localiser l'artère radiale 11 dans le poignet 12 d'un patient humain.

Bien entendu, selon d'autres modes de réalisation, la pince ou même tout autre dispositif de localisation conforme à l'invention peut être utilisé pour localiser tout vaisseau sanguin tels que des capillaires ou des veines ou des artères pour tout être vivant tel qu'un être humain ou un animal. De même, ils peuvent être utilisé pour une telle détection dans tout membre tel que par exemple le poignet, le bras, l'aine, et la jambe ou la patte.

Dans le présent mode de réalisation de l'invention, le dispositif de localisation 10 (par exemple la ponce 10) est utilisée par un soignant ou praticien dans le cadre d'une prise de sang artérielle pour effectuer une analyse du gaz du sang du patient. Ainsi, dans le présent exemple, l'artère cible est l'artère radiale 11 du poignet 12 du patient.

Par exemple, le dispositif de localisation 10 comprend des moyens de détermination 1 de la localisation de l'artère radiale 11 (ci-après « moyens de détermination 1 »).

Préférentiellement, le dispositif de localisation 10 est disposé sur le poignet 12 du patient de sorte que des moyens de détermination 1 du dispositif de localisation 10 soient placés en regard de la face intérieure du poignet 12. Par exemple, le dispositif de localisation 10 comprend une première branche 41 comprenant les moyens de détermination 1. Par exemple, la première branche 41 est prévue pour être positionnée de sorte à mettre en regard les moyens de détermination 1 et une surface de peau recouvrant le vaisseau sanguin du membre. Par exemple, le dispositif de localisation 10 comprend également une seconde branche 42 prévue pour maintenir la première branche 41 contre le membre. Par exemple, le dispositif de localisation 10 comprend des moyens de mise en mouvement relatif de la première branche 41 par rapport à la seconde branche 42.

Par exemple, le dispositif de localisation 10 comprend des moyens d'alimentation électrique des moyens de détermination 1 de la localisation de l'artère 11. Par exemple, ces moyens d'alimentation sont une batterie rechargeable. Par exemple, la batterie est positionnée dans la première branche. Bien entendu, la batterie ou plus généralement, tout moyen d'alimentation électrique du dispositif de localisation 10 ou des moyens de détermination 1 du dispositif de localisation 10 peuvent être localisé en toute partie du dispositif de localisation 10 (par exemple dans la seconde branche ou même ailleurs) ou même en dehors du dispositif de localisation 10 et dans ce cas l'énergie électrique peut être amenée au dispositif de localisation 10 par tout moyen tel qu'un câble ou fil électrique ou par induction ou par tout autre moyen.

Par exemple, au moins une des première et seconde branche présente une forme sensiblement en arc de cercle de sorte à épouser la morphologie du membre. Par exemple, chacune des première et seconde branche présente une forme sensiblement en arc de cercle et épouse donc le poignet du patient. Par exemple, dans le dispositif de localisation 10, les moyens de mise en mouvement comprennent des moyens de translation 1000 de la première branche 41 par rapport à la seconde branche 42.

Selon un autre exemple non illustré, dans le dispositif de localisation 10, les moyens de mise en mouvement comprennent des moyens d'articulation de la première branche 41 par rapport à la seconde branche 42.

Par exemple, le dispositif de localisation 10 comprend des moyens de motorisation des moyens de mise en mouvement. Par exemple, les moyens de motorisation des moyens de mise en mouvement comprennent un système à base de pression pneumatique. Bien entendu, tout autre système de pression (par exemple pression hydraulique) ou tout autre système de motorisation peut être mis en œuvre.

Selon un mode de réalisation de la présente invention, la première branche 41 comprend un premier doigt 411 en regard d'une première portion de peau du poignet et un second doigt 412 en regard d'une seconde portion de peau du poignet.

Selon un mode de réalisation de l'invention, les moyens de détermination 1 de la localisation de l'artère 11 comprennent au moins un émetteur d'ondes acoustiques configuré pour émettre des ondes acoustiques - ci-après onde acoustiques émises - dans le membre à travers la surface de peau, au moins un récepteur d'ondes acoustiques configuré pour recevoir les ondes acoustiques réfléchies par le membre - ci-après ondes acoustiques réfléchies, un module de mesure d'un paramètre des ondes acoustiques réfléchies ou d'une différence de valeur d'un paramètre entre les ondes acoustiques réfléchies et les ondes acoustiques émises et un module d'obtention de la localisation du vaisseau sanguin en fonction dudit paramètre ou de ladite différence de valeur dudit paramètre.

Bien entendu, les moyens de détermination 1 peuvent mettre en œuvre toute autre technologie de localisation de vaisseau sanguin. Par exemple, ils peuvent mettre en œuvre des ondes optiques émises et reçues (par exemple, en émission par des diodes, lasers ou toutes autres sources de lumière et, en réception, par des photodiodes ou tout autre dispositifs de détection de lumière). Par exemple encore, ils peuvent mettre en œuvre de la détection par mesure de pression, par émission d'ondes radio fréquence ou toute autre technologie. Par exemple encore, ils peuvent ne mettre en œuvre que des moyens de détection d'ondes optiques, acoustiques, radiofréquence ou pression sans moyens d'émission de telles ondes ou pression.

Les moyens de détermination 1 sont configurés pour être disposés sensiblement en regard d'une surface de peau recouvrant l'artère du poignet. Selon un mode de réalisation de l'invention, les moyens de détermination 1 sont configurés pour être disposés sensiblement en contact d'une surface de peau recouvrant l'artère du poignet. Cependant, selon des variantes de ce mode de réalisation de l'invention, les moyens de détermination 1 peuvent être prévus pour être disposés à distance (par exemple à quelques millimètres ou à quelques centimètres) de la surface de peau.

Par exemple, le premier doigt 411 comprend au moins un premier émetteur d'ondes acoustiques et au moins un premier récepteur d'ondes acoustiques et le second doigt 412 comprend au moins un second émetteur d'ondes acoustiques et au moins un second récepteur d'ondes acoustiques.

Par exemple, les moyens de détermination 1 comprennent au moins un émetteur d'ondes acoustiques 2, 21, 22 configuré pour émettre des ondes acoustiques (ci-après ondes acoustiques émises) dans le membre à travers la surface de peau, au moins un récepteur d'ondes acoustiques 3, 31, 32 configuré pour recevoir les ondes acoustiques réfléchies par le poignet 12 (ci-après ondes acoustiques réfléchies).

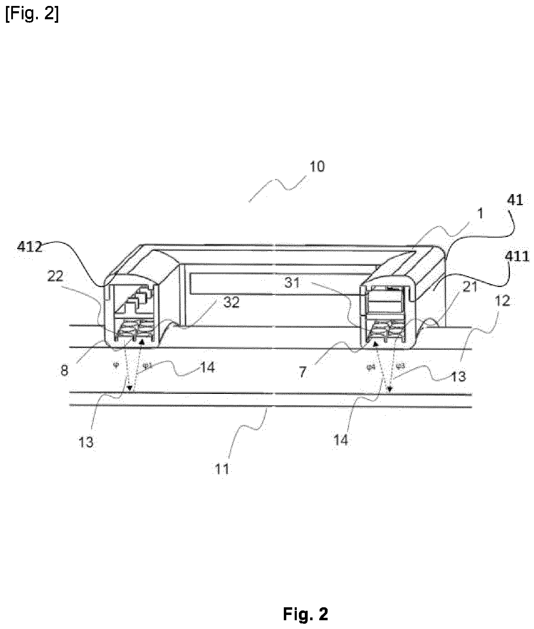

Par exemple et tel qu'illustré par la figure 2, les moyens de détermination 1 comprennent une première matrice 7 d'émetteurs 21 et de récepteurs 31 d'ondes acoustiques située dans le premier doigt 411 et une seconde matrice 8 d'émetteurs 22 et de récepteurs 32 d'ondes acoustiques située dans le second doigt 412.

Bien entendu, selon des variantes de l'invention, les moyens de détermination 1 peuvent comprendre dans le premier doigt 411, un nombre quelconque d'émetteurs et de récepteurs d'ondes acoustiques organisés en matrice ou non et ils peuvent également comprendre dans le second doigt 412, un nombre quelconque d'émetteurs et de récepteurs d'ondes acoustiques organisés en matrice ou non.

Par exemple, les moyens de détermination 1 présentent en vue de dessus une forme sensiblement carrée ou rectangulaire. Préférentiellement la distance entre la première matrice 7 dans le premier doigt et la seconde matrice 8 dans le second doigt fait moins de 2cm et encore préférentiellement moins de 1,5cm afin que l'artère puisse être considérée comme sensiblement rectiligne entre la première matrice 7 et la seconde matrice 8.

Selon un mode de réalisation, chaque émetteur 2, 21, 22 et récepteur 3, 31, 32 d'ondes acoustiques peut être reconfiguré respectivement en un récepteur et/ou un émetteur d'ondes acoustiques. Bien entendu, selon des variantes de ce mode de réalisation, seule une partie des émetteurs et/ou récepteurs (par exemple au moins un d'entre eux) peut être reconfigurée. Selon encore une autre variante, aucun des récepteurs ou émetteurs n'est reconfigurable.

Par exemple, chacun des émetteurs et des récepteurs d'onde acoustiques comprend un élément piézoélectrique, par exemple un disque piézoélectrique dont le diamètre est par exemple compris entre 0,5mm et 1cm et plus préférentiellement encore compris entre 1mm et 3mm. Par exemple, chacun des disques piézoélectrique présente un diamètre de 1 mm. Bien entendu, l'invention peut être implémentée avec toute autre valeur de diamètre des disques piézoélectriques. Par ailleurs, toute autre forme d'élément piézoélectrique peut être mis en œuvre dans le cadre de l'invention. Par ailleurs, d'autres types d'émetteurs et récepteurs que les solutions à piézoélectriques peuvent être mis en œuvre dans le cadre de l'invention.

Par exemple, les moyens de détermination 1 du dispositif de localisation 10 comprennent également des moyens de contrôle des émetteurs et récepteurs. Ces moyens de contrôle sont par exemple réalisés sous la forme d'un circuit électronique. Ainsi, selon une implémentation préférentielle de l'invention, le circuit électronique peut alimenter certains des émetteurs et récepteurs des première 7 et seconde 8 matrices selon une séquence déterminée. Par exemple, en se référant à la figure 2 qui montre schématiquement le dispositif de localisation 10 installée sur le poignet 12 et le cheminement des ondes acoustiques émises 13 et réfléchies 14, une telle séquence peut comprendre la mise en oeuvre simultanée des quatre étapes suivantes :
- configurer en émetteur le premier disque piézoélectrique de la première matrice en partant de la gauche sur la ligne extérieure de la première matrice (la ligne de disques piézoélectriques la plus éloignée du centre des moyens de détermination 1) ;
- configurer en récepteur le premier disque piézoélectrique de la première matrice en partant de la gauche sur la ligne intérieure de la première matrice (la ligne de disques piézoélectriques la plus proche du centre des moyens de détermination 1) ;
- configurer en émetteur le premier disque piézoélectrique de la seconde matrice en partant de la gauche sur la ligne intérieure de la seconde matrice (la ligne de disques piézoélectriques la plus proche du centre des moyens de détermination 1) ;
- configurer en récepteur le premier disque piézoélectrique de la seconde matrice en partant de la gauche sur la ligne intérieure de la seconde matrice (la ligne de disques piézoélectriques la plus éloignée du centre des moyens de détermination 1).

Puis, la mise en œuvre simultanée des mêmes quatre étapes avec le second disque piézoélectrique de chaque ligne de chaque matrice et ainsi de suite de sorte à balayer horizontalement tous les disques piézoélectrique des première matrice et seconde matrice.

Un tel balayage permet de mesurer les ondes acoustiques réfléchies 14 par le poignet 12 sur toute la longueur des matrices de disques piézoélectrique.

Par exemple, la longueur des première et seconde matrice est identique et l'espacement entre les disques dans chaque ligne est également identique. Ainsi, par exemple, chaque ligne de chaque matrice comprend le même nombre de disques piézoélectriques. Par exemple, la longueur de la première matrice 7 et de la seconde matrice 8 est prévue dans les moyens de détermination 1 afin que chaque matrice dans le dispositif surplombe l'artère 11 dans le poignet 12.

Le circuit électronique comprend un module de mesure d'un paramètre des ondes acoustiques réfléchies 14 ou d'une différence de valeur d'un paramètre entre les ondes acoustiques réfléchies 14 et les ondes acoustiques émises 13.

Le circuit électronique comprend également un module de d'obtention de la localisation du vaisseau sanguin en fonction du paramètre ou de la différence de valeur du paramètre.

Préférentiellement, le module de mesure du paramètre des ondes acoustiques réfléchies 14 ou d'une différence de valeur d'un paramètre entre les ondes acoustiques réfléchies 14 et les ondes acoustiques émises 13 comprend un filtre et un amplificateur.

Selon un premier mode de réalisation de l'invention, le module de mesure est un module de mesure du déphasage entre les ondes acoustiques réfléchies et les ondes acoustiques émises.

Selon un second mode de réalisation de l'invention, le module de mesure est un module de mesure de l'amplitude des ondes acoustiques réfléchies. Selon un troisième mode de réalisation de l'invention, le module de mesure est un module de mesure de la différence de fréquence entre les ondes acoustiques réfléchies et les ondes acoustiques émises.

Par exemple, on va mesurer le déphasage entre les ondes acoustiques émises et les ondes acoustiques réfléchies en fonction du temps t sur la durée d'un balayage d'une matrice de disques piézoélectriques de la pince 10 dans le cadre du premier mode de réalisation précité. Le déphasage Δf est égale à la différence de phase Phi 1 - Phi entre les ondes réfléchies 14 et les ondes émises 13.

Ainsi, par exemple, lors du balayage des matrices de disques piézoélectriques mis en oeuvre par le circuit électronique précédemment décrit, les moyens de mesure du déphasage vont mesurer le déphasage entre les ondes émises 13 et les ondes réfléchies 14 au niveau de chaque couple de disque piézoélectrique sur les deux lignes de chaque matrice.

Ainsi, pour chacune de la première matrice 7 et seconde matrice 8, un balayage est par exemple mis en œuvre.

Dans une matrice 7, 8 donnée, lorsque les ondes acoustiques émises atteignent ou se rapprochent de l'artère 11, le déphasage entre les ondes émises et les ondes réfléchies va présenter une ou des valeur(s) maximale(s) qui est/sont caractéristique(s) de la présence de l'artère (en générale, on va obtenir un couple de valeurs maximales qui indique que l'artère se situe entre les couples de disques concernés). Ainsi, le circuit électronique des moyens de détermination 1 peut identifier à partir du déphasage mesuré des valeurs maximales qui sont caractéristiques de la présence de l'artère sous les couples de disques piézoélectriques concernés dans le poignet.

Dans le cadre du second mode de réalisation, l'amplitude des ondes réfléchies est mesurée à la place du déphasage et, de la même manière, la valeur maximum de l'amplitude est caractéristique de la présence de l'artère.

Dans le cadre du troisième mode de réalisation, la différence de fréquence entre les ondes réfléchies et les ondes émise est mesurée à la place du déphasage et, de la même manière, la valeur maximum de la différence de fréquence est caractéristique de la présence de l'artère.

Le circuit électronique comprend également un module d'obtention de la localisation de l'artère en fonction du déphasage mesuré dans le premier mode de réalisation précité (ou en fonction de de l'amplitude des ondes acoustiques réfléchies dans le second mode de réalisation précité ou encore en fonction de la différence de fréquence entre les ondes réfléchies et les ondes émises dans le troisième mode de réalisation précité).

Ce module va donc identifier le maximum de déphasage mesuré par le module de mesure du déphasage dans le premier mode de réalisation (ou le maximum d'amplitude des ondes acoustiques réfléchies dans le second mode de réalisation ou le maximum de différence de fréquence dans le troisième mode de réalisation) lors du balayage sur chacune des première et seconde matrice, ce qui lui permet de déterminer quels couples de disques piézoélectriques sont concerné par le maximum au niveau de chacune des première 7 et seconde 8 matrices.

Ceci lui permet donc de localiser l'artère 11 en deux points : l'un au niveau de la première matrice et l'autre au niveau de la seconde matrice. Puis, par exemple, le module d'obtention de la localisation de l'artère 11 opère une interpolation linéaire entre les deux points et détermine une localisation d'une portion de l'artère 11 entre ces deux points.

Ainsi, tel que décrit précédemment, le dispositif de localisation 10 et notamment ses moyens de détermination 1 mettent en œuvre :
- un balayage par les émetteurs et récepteur de la première matrice 7 afin d'identifier les deux couples d'émetteurs / récepteurs de cette dernière qui présentent la réponse (par exemple le déphasage) la plus importante (l'artère 11 se situe donc entre ces deux couples). Une fois ces deux couples identifiés, par exemple en fonction du délai et de la force de réception entre ces deux couples, le dispositif peut calculer la distance entre l'artère 11 et chaque couple. Grace à ces informations il est possible de connaitre la position de l'artère dans le référentiel de la première matrice 7 (localisation d'un premier point de l'artère).
- un même balayage est réalisé avec la seconde matrice 8 ce qui permet d'obtenir la localisation d'un second point de l'artère.

Selon le présent mode de réalisation, le dispositif de localisation 10 comprend également des moyens de projection 100 sur la surface de peau d'au moins une image 50 de localisation de l'artère 11.

Par exemple, une image 50 de localisation de l'artère 11 comprend au moins une des images suivantes :
- une information représentative de la profondeur de l'artère dans le poignet 12 ;
- une représentation d'une portion de l'artère.

Par exemple la ou les image(s) de localisation 50 est/sont projetée(s) sur la surface de peau de manière à ce qu'elle(s) se situe(nt) sensiblement à l'aplomb de l'artère 11.

Par exemple, les moyens de projection 100 comprennent au moins un projecteur et un miroir inclinable.

Selon un mode de réalisation de l'invention, une image 50 de localisation de l'artère 11 est une portion de l'artère 11 qui est construite à partir d'une première localisation déterminée à partir de la localisation de l'artère 11 en deux points au moyen des première et seconde matrices 7 et 8 tel qu'indiqué ci-dessus.

Par exemple, une image de localisation 50 selon l'invention comprend également un nombre (ou même toute autres information représentative de la profondeur) disposé à proximité (dans l'image de localisation 50) de la portion de l'artère 11 (mentionnée ci-dessus) qui est la profondeur de l'artère dans le poignet.

Bien entendu, selon des variantes de ce mode de réalisation, l'image 50 peut ne comprendre que la profondeur ou alors qu'une portion de l'artère.

Ainsi, les informations de localisation (par exemple les localisations des premier et second points mentionnées ci-dessus) sont transmises aux moyens de projection de l'image.

Au niveau des moyens de projection de l'image, un programme va transposer les informations de localisation de l'artère 11 obtenues dans une matrice virtuelle représentant une zone d'affichage (dans laquelle est affichée l'image) en lui attribuant des coordonnées. En effet, du fait que la première matrice 7 et la seconde matrice 8 sont fixes par rapport à la zone d'affichage les informations de localisation y sont transposées.

Une fois ces informations de localisation (par exemple les premier et second points mentionnés ci-dessus) transposés dans la zone d'affichage, le dispositif de localisation trace une droite entre ces deux points. Cette droite représente le parcours de l'artère radiale dans la zone de travail. Une fois cela effectué l'image constituée est projetée sur le poignet

Pour cela un projecteur est placé dans la partie supérieure du dispositif et un renvoi d'angle par miroir est effectué pour rediriger l'image dans la zone de projection finale.

Selon l'invention, le dispositif de localisation 10 comprend des moyens de détection d'un déplacement de l'artère 11. Par exemple, les moyens de détermination 1 de la localisation de l'artère 11 et les moyens de projection 100 sont activés par les moyens de détection d'un déplacement de l'artère 11.

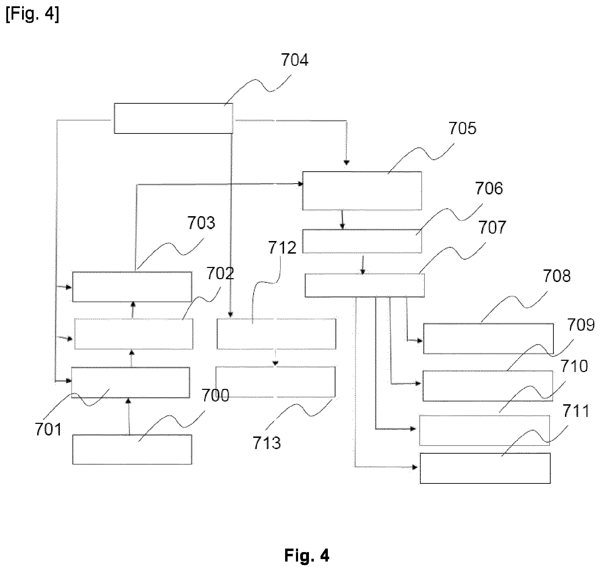

Ainsi, selon un mode de réalisation de l'invention, il peut être mis en œuvre une boucle qui a pour objectif de corriger tout déplacement de l'artère pas par rapport à l'image projetée sur le poignet par exemple lors de l'intervention par le praticien sur l'artère (durant le geste). Par exemple, selon une telle boucle, si l'artère ne se déplace pas, alors aucune correction de l'image n'est opérée. En revanche, si le dispositif détecte un déplacement de l'artère par rapport à l'image, alors la localisation de l'artère est à nouveau recherchée (tel que décrit ci-dessus) à l'aide des première et seconde matrices (par exemple en se basant sur les trois couples d'émetteurs / récepteurs générant la plus forte réponse).. De cette manière, une nouvelle image représentative de la nouvelle localisation de l'artère est projetée.. Cela permet d'effectuer un suivi de l'artère radiale en permanence et d'actualiser les informations fournies à l'opérateur. La figure 4 montre est schéma bloc du circuit électronique de la pince 10 de la figure 1.

Un signal acoustique 700 (réfléchie sur le poignet) est reçu par un disque piézoélectrique configuré en récepteur 701. Le signal électrique généré en conséquence par le disque piézoélectrique (récepteur) est ensuite transmis à un filtre parasite 702 qui transmet ensuite le signal filtré à un amplificateur 703. Le filtre parasite 702 est par exemple un filtre passe haut actif du troisième ordre qui présente par exemple un gain de 24 dB (obtenu par la formule suivante : Gain = 33000/1500+22000/9100 -= 24dB).

Par exemple l'amplificateur 703 est un amplificateur AD847 qui présente un produit gain par bande passante de l'ordre de 50MHz. L'amplificateur 703 transmet ensuite le signal amplifié à un module de traitement de données 705 (qui comprend le module de mesure de paramètre ou de différence de paramètre et le module de détermination de la localisation précédemment mentionnés). Le module de traitement des données 705 transmet un signal à un module de commande 706 qui commande un module d'alimentation 707 qui lui-même alimente une commande moteur de l'actionneur du premier laser 91, une commande moteur de l'actionneur du second laser 92 ainsi que les premier 91 et second 92 laser. Une alimentation 704 alimente les disque piézoélectriques émetteurs et récepteurs, le filtre parasite 702 et l'amplificateur 703.

Par exemple l'alimentation 704 alimente les disques piézoélectrique lorsque configurés en émetteurs avec un signal à 3 MHz.

L'invention concerne en particulier aussi un procédé pour la fabrication/le montage d'un dispositif de localisation d'un vaisseau sanguin.

### Liste de signes de référence

- 1: moyens de localisation
- 2: émetteur d'ondes acoustiques
- 21: émetteur d'ondes acoustiques
- 22: émetteur d'ondes acoustiques
- 3: récepteur d'ondes acoustiques
- 31: récepteur d'ondes acoustiques
- 32: récepteur d'ondes acoustiques
- 41: première branche
- 411: premier doigt
- 412: second doigt
- 42: seconde branche
- 50: image
- 7: première matrice
- 8: seconde matrice
- 10: dispositif de localisation
- 100: moyens de projection
- 11: Artère
- 12: poignet
- 13: ondes acoustiques émises
- 14: ondes acoustiques réfléchies
- 1000: moyens de translation

## Revendications

1. Dispositif de localisation (10) d'un vaisseau sanguin d'un membre d'un être vivant configuré pour être disposé sensiblement en regard d'une surface de peau recouvrant le vaisseau sanguin (11) dudit membre, le dispositif (10) comprenant des moyens de détermination (1) de la localisation du vaisseau sanguin, le dispositif de localisation comprenant des moyens de projection (100) sur la surface de peau d'au moins une image (50) de localisation du vaisseau sanguin (11), ladite au moins une image (50) de localisation du vaisseau sanguin (11) comprend :
- une information représentative de la profondeur du vaisseau sanguin dans ledit membre et
- une représentation d'une portion du vaisseau sanguin,
**caractérisé en ce que** ledit dispositif de localisation (10) comprend des moyens de détection d'un déplacement du vaisseau sanguin,
et **en ce que** lesdits moyens de détermination (1) de la localisation du vaisseau sanguin et les moyens de projection (100) d'au moins une image de localisation sont activés par les moyens de détection d'un déplacement du vaisseau sanguin (11).

2. Dispositif de localisation (10) d'un vaisseau sanguin selon la revendication précédente, **caractérisé en ce que** ladite au moins une image (50) est projetée sur la surface de peau de manière à ce qu'elle(s) se situe(nt) sensiblement à l'aplomb du vaisseau sanguin (11).

3. Dispositif de localisation (10) d'un vaisseau sanguin selon l'une quelconque des revendications précédentes, **caractérisé en ce que** les moyens de projection (100) comprennent au moins un projecteur et un miroir inclinable.

4. Dispositif de localisation (10) d'un vaisseau sanguin selon l'une quelconque des revendications précédentes, **caractérisé en ce que** les moyens de détermination (1) de la localisation du vaisseau sanguin (11) comprennent au moins un émetteur d'ondes acoustiques (2, 21, 22) configuré pour émettre des ondes acoustiques - ci-après onde acoustiques émises - dans le membre à travers la surface de peau, au moins un récepteur d'ondes acoustiques (3, 31, 32) configuré pour recevoir les ondes acoustiques réfléchies par le membre - ci-après ondes acoustiques réfléchies, un module de mesure d'un paramètre des ondes acoustiques réfléchies ou d'une différence de valeur d'un paramètre entre les ondes acoustiques réfléchies et les ondes acoustiques émises et un module d'obtention de la localisation du vaisseau sanguin en fonction dudit paramètre ou de ladite différence de valeur dudit paramètre.

5. Dispositif de localisation (10) d'un vaisseau sanguin selon la revendication 4, **caractérisé en ce que** les moyens de détermination (1) de la localisation du vaisseau sanguin comprennent au moins une première matrice (7) d'émetteurs et de récepteurs d'ondes acoustiques située en une première zone du dispositif et une seconde matrice (8) d'émetteurs et de récepteurs d'ondes acoustiques située en une seconde zone du dispositif.

6. Dispositif de localisation (10) d'un vaisseau sanguin selon la revendication 5, **caractérisé en ce que** le module de mesure d'un paramètre ou d'une différence de valeur d'un paramètre comprend des moyens de mesure d'une première valeur de paramètre ou de différence de valeur de paramètre au niveau de la première zone et des moyens de mesure d'une seconde valeur de paramètre ou de différence de valeur de paramètre au niveau de la seconde zone,
et **en ce qu'**une image (50) de localisation du vaisseau sanguin (11) est construite à partir d'une première localisation déterminée à partir de la première valeur de paramètre ou de différence de valeur de paramètre et d'une seconde localisation déterminée à partir de la seconde valeur de paramètre ou de différence de valeur de paramètre.

7. Dispositif de localisation (10) d'un vaisseau sanguin selon l'une quelconque des revendications 5 et 6, **caractérisé en ce qu'**il comprend une première branche (41) comprenant les moyens de détermination de la localisation (1) du vaisseau sanguin, ladite première branche étant prévue pour être positionnée de sorte à mettre en regard les moyens de détermination de la localisation et la surface de peau recouvrant le vaisseau sanguin dudit membre et une seconde branche prévue pour maintenir la première branche contre ledit membre, et **en ce que** la première branche comprend un premier doigt comprenant la première matrice (7) et un second doigt comprenant la seconde matrice (8).

8. Dispositif de localisation (10) d'un vaisseau sanguin selon l'une quelconque des revendications précédentes, **caractérisé en ce que** le dispositif de localisation est une pince (10) adaptée pour être disposée sur le membre d'un être vivant.

9. Procédé de localisation d'un vaisseau sanguin (11) d'un membre d'un être vivant configuré pour être disposé sensiblement en regard d'une surface de peau recouvrant le vaisseau sanguin (11) dudit membre, le procédé comprenant une étape de détermination de la localisation du vaisseau sanguin, ledit procédé de localisation comprenant une étape de projection sur la surface de peau d'au moins une image (50) de localisation du vaisseau sanguin (11),
ladite au moins une image (50) de localisation du vaisseau sanguin comprenant :
- une information représentative de la profondeur du vaisseau sanguin (11) dans ledit membre et
- une représentation d'une portion du vaisseau sanguin (11),
ledit procédé étant **caractérisé en ce qu'**il comprend une étape de détection d'un déplacement du vaisseau sanguin (11) et **en ce que** la détection d'un déplacement du vaisseau sanguin (11) active une étape de détermination de la localisation du vaisseau sanguin (11) et une étape de projection sur la surface de peau d'au moins une image (50) de localisation du vaisseau sanguin (11).

10. Procédé de localisation selon la revendication 9, **caractérisé en ce que** ladite au moins une image (50) est projetée sur la surface de peau de manière à ce qu'elle(s) se situe(nt) sensiblement à l'aplomb du vaisseau sanguin (11).

## Patentansprüche

1. Lokalisierungsvorrichtung (10) eines Blutgefäßes eines Körperglieds eines Lebewesens, die konfiguriert ist, um im Wesentlichen gegenüber einer Hautoberfläche angeordnet zu sein, die das Blutgefäß (11) des Körperglieds bedeckt, wobei die Vorrichtung (10) Bestimmungseinrichtungen (1) der Lokalisierung des Blutgefäßes umfasst, die Lokalisierungsvorrichtung Projektionseinrichtungen (100) von mindestens einem Bild (50) der Lokalisierung des Blutgefäßes (11) auf der Hautoberfläche umfasst, wobei das mindestens eine Bild (50) der Lokalisierung des Blutgefäßes (11) Folgendes umfasst:
- Informationen, welche die Tiefe des Blutgefäßes in dem Körperglied darstellen, und
- eine Darstellung eines Abschnitts des Blutgefäßes,
**dadurch gekennzeichnet, dass** die Lokalisierungsvorrichtung (10) Erfassungseinrichtungen einer Bewegung des Blutgefäßes umfasst,
und dadurch, dass die Bestimmungseinrichtungen (1) der Lokalisierung des Blutgefäßes und die Projektionseinrichtungen (100) von mindestens einem Bild der Lokalisierung durch die Erfassungseinrichtungen einer Bewegung des Blutgefäßes (11) aktiviert werden.

2. Lokalisierungsvorrichtung (10) eines Blutgefäßes nach dem vorhergehenden Anspruch, **dadurch gekennzeichnet, dass** das mindestens eine Bild (50) auf die Hautoberfläche projiziert wird, sodass es/sie im Wesentlichen lotrecht zu dem Blutgefäß (11) ist/sind.

3. Lokalisierungsvorrichtung (10) eines Blutgefäßes nach einem der vorhergehenden Ansprüche, **dadurch gekennzeichnet, dass** die Projektionseinrichtungen (100) mindestens einen Projektor und einen neigbaren Spiegel umfassen.

4. Lokalisierungsvorrichtung (10) eines Blutgefäßes nach einem der vorhergehenden Ansprüche, **dadurch gekennzeichnet, dass** die Bestimmungseinrichtungen (1) der Lokalisierung des Blutgefäßes (11) mindestens einen Sender akustischer Wellen (2, 21, 22), der konfiguriert ist, um akustische Wellen - im Folgenden emittierte akustische Welle - durch die Hautoberfläche in das Körperglied zu emittieren, mindestens einen Empfänger akustischer Wellen (3, 31, 32), der konfiguriert ist, um die von dem Körperglied reflektierten akustischen Wellen - im Folgenden reflektierte akustische Wellen - zu empfangen, ein Messmodul eines Parameters der reflektierten akustischen Wellen oder einer Wertdifferenz eines Parameters zwischen den reflektierten akustischen Wellen und den emittierten akustischen Wellen und ein Erlangungsmodul der Lokalisierung des Blutgefäßes abhängig von dem Parameter oder der Wertdifferenz des Parameters umfassen.

5. Lokalisierungsvorrichtung (10) eines Blutgefäßes nach Anspruch 4, **dadurch gekennzeichnet, dass** die Bestimmungseinrichtungen (1) der Lokalisierung des Blutgefäßes mindestens eine erste Matrix (7) aus Sendern und Empfängern akustischer Wellen, die sich in einem ersten Bereich der Vorrichtung befindet, und eine zweite Matrix (8) aus Sendern und Empfängern akustischer Wellen, die sich in einem zweiten Bereich der Vorrichtung befindet, umfassen.

6. Lokalisierungsvorrichtung (10) eines Blutgefäßes nach Anspruch 5, **dadurch gekennzeichnet, dass** das Messmodul eines Parameters oder einer Wertdifferenz eines Parameters Messeinrichtungen eines ersten Parameterwerts oder einer Parameterwertdifferenz auf Ebene des ersten Bereichs und Messeinrichtungen eines zweiten Parameterwerts oder einer Parameterwertdifferenz auf Ebene des zweiten Bereichs umfasst,
und dadurch, dass ein Bild (50) der Lokalisierung des Blutgefäßes (11) aus einer ersten Lokalisierung, die anhand des ersten Parameterwerts oder der Parameterwertdifferenz bestimmt wird, und einer zweiten Lokalisierung, die anhand des zweiten Parameterwerts oder der Parameterwertdifferenz bestimmt wird, aufgebaut wird.

7. Lokalisierungsvorrichtung (10) eines Blutgefäßes nach einem der Ansprüche 5 und 6, **dadurch gekennzeichnet, dass** sie einen ersten Zweig (41) umfasst, umfassend die Bestimmungseinrichtungen der Lokalisierung (1) des Blutgefäßes, wobei der erste Zweig bereitgestellt ist, um positioniert zu werden, um die Bestimmungseinrichtungen der Lokalisierung und die Hautoberfläche, die das Blutgefäß des Körperglieds bedeckt, einander gegenüberzustellen, und einen zweiten Zweig, der bereitgestellt ist, um den ersten Zweig gegen das Körperglied zu halten, und dadurch, dass der erste Zweig einen ersten Finger, umfassend die erste Matrix (7), und einen zweiten Finger, umfassend die zweite Matrix (8), umfasst.

8. Lokalisierungsvorrichtung (10) eines Blutgefäßes nach einem der vorhergehenden Ansprüche, **dadurch gekennzeichnet, dass** die Lokalisierungsvorrichtung eine Klammer (10) ist, die angepasst ist, um an dem Körperglied eines Lebewesens angeordnet zu werden.

9. Verfahren zur Lokalisierung eines Blutgefäßes (11) eines Körperglieds eines Lebewesens, das konfiguriert ist, um im Wesentlichen gegenüber einer Hautoberfläche angeordnet zu sein, die das Blutgefäß (11) des Körperglieds bedeckt, wobei das Verfahren einen Schritt des Bestimmens der Lokalisierung des Blutgefäßes umfasst, wobei das Lokalisierungsverfahren einen Schritt des Projizierens von mindestens einem Bild (50) der Lokalisierung des Blutgefäßes (11) auf die Hautoberfläche umfasst,
wobei das mindestens eine Bild (50) der Lokalisierung des Blutgefäßes Folgendes umfasst:
- Informationen, welche die Tiefe des Blutgefäßes (11) in dem Körperglied darstellen, und
- eine Darstellung eines Abschnitts des Blutgefäßes (11),
wobei das Verfahren **dadurch gekennzeichnet ist, dass** es einen Schritt des Erfassens einer Bewegung des Blutgefäßes (11) umfasst, und dadurch, dass das Erfassen einer Bewegung des Blutgefäßes (11) einen Schritt des Bestimmens der Lokalisierung des Blutgefäßes (11) und einen Schritt des Projizierens von mindestens einem Bild (50) der Lokalisierung des Blutgefäßes (11) auf die Hautoberfläche aktiviert.

10. Lokalisierungsverfahren nach Anspruch 9, **dadurch gekennzeichnet, dass** das mindestens eine Bild (50) auf die Hautoberfläche projiziert wird, sodass es/sie im Wesentlichen lotrecht zu dem Blutgefäß (11) ist/sind.

## Claims

1. Device (10) for locating a blood vessel in a limb of a living being, configured to be arranged substantially opposite a surface of skin covering the blood vessel (11) of said limb, the device (10) comprising means (1) for determining the location of the blood vessel, the locating device comprising means (100) for projecting, onto the surface of the skin, at least one image (50) for locating the blood vessel (11), said at least one image (50) for locating the blood vessel (11) comprises:
- information representative of the depth of the blood vessel in said limb, and
- a representation of a portion of the blood vessel, **characterized in that** said location device (10) comprises means for detecting a movement of the blood vessel,
and **in that** said means (1) for determining the location of the blood vessel and the means (100) for projecting at least one locating image are activated by the means for detecting a movement of the blood vessel (11).

2. Device (10) for locating a blood vessel according to the preceding claim, **characterised in that** said at least one image (50) is projected onto the surface of the skin, such that it/they is/are located substantially in vertical alignment with the blood vessel (11).

3. Device (10) for locating a blood vessel according to any one of the preceding claims, **characterised in that** the projection means (100) comprise at least one projector and an inclinable mirror.

4. Device (10) for locating a blood vessel according to any one of the preceding claims, **characterised in that** the means (1) for determining the location of the blood vessel (11) comprise at least one acoustic wave transmitter (2, 21, 22), configured to transmit acoustic waves - hereinafter, transmitted acoustic waves - into the limb through the surface of the skin, at least one acoustic wave receiver (3, 31, 32), configured to receive the acoustic waves reflected by the limb - hereinafter, reflected acoustic waves, a module for measuring a parameter of the reflected acoustic waves or a difference in value of a parameter between the reflected acoustic waves and the transmitted acoustic waves and module for obtaining the location of the blood vessel, as a function of said parameter or of said difference in value of said parameter.

5. Device (10) for locating a blood vessel according to claim 4, **characterised in that** the means (1) for determining the location of the blood vessel comprise at least one first acoustic wave transmitter and receiver matrix (7), located in a first zone of the device and a second zone of the device and a second acoustic wave transmitter and receiver matrix (8), located in a second zone of the device.

6. Device (10) for locating a blood vessel according to claim 5, **characterised in that** the module for measuring a parameter of a difference in value of a parameter comprises means for measuring a first parameter value or difference in parameter value at the first zone, and means for measuring a second parameter value or difference in parameter value at the second zone,
and **in that** an image (50) for locating the blood vessel (11) is constructed from a first location determined from the first parameter value or different in parameter value and from a second location determined from the second parameter value or different in parameter value.

7. Device (10) for locating a blood vessel according to any one of claims 5 and 6, **characterised in that** it comprises a first branch (41) comprising the means (1) for determining the location of the blood vessel, said first branch being provided to be positioned, so as to make the means for determining the location and the surface of the skin covering the blood vessel of said limb face one another, and a second branch provided to hold the first branch against said limb, and **in that** the first branch comprises a first finger comprising the first matrix (7) and a second finger comprising the second matrix (8).

8. Device (10) for locating a blood vessel according to any one of the preceding claims, **characterised in that** the locating device is a clamp (10) adapted to be arranged on the limb of a living being.

9. Method for locating a blood vessel (11) in a limb of a living being, configured to be arranged substantially opposite a surface of skin covering the blood vessel (11) of said limb, the method comprising a step of determining the location of the blood vessel, said locating method comprising a step of projecting, onto the surface of the skin, at least one image (50) for locating the blood vessel (11),
said at least one image (50) for locating the blood vessel comprising:
- information representative of the depth of the blood vessel (11) in said limb, and
- a representation of a portion of the blood vessel (11),
said method being **characterised in that** it comprises a step of detecting a movement of the blood vessel (11), and **in that** the detection of a movement of the blood vessel (11) activates a step of determining the location of the blood vessel (11) and a step of projecting, onto the surface of the skin, at least one image (50) for locating the blood vessel (11).

10. Locating method according to claim 9, **characterised in that** said at least one image (50) is projected onto the surface of the skin, such that it/they is/are located substantially in vertical alignment with the blood vessel (11).
